# FASCICULE DE BREVET EUROPEEN

(11) **EP 0 478 418 B1**
(45) Date de publication et mention de la délivrance du brevet: **10.01.1996**
(21) Numéro de dépôt: 91402475.7
(22) Date de dépôt: 17.09.1991
(51) Int. Cl.: C07C 11/02, C07C 1/20, B01J 8/18

(54) **Procédé catalytique pour la préparation d'olefines**
Katalytisches Verfahren zur Herstellung von Olefinen
Catalytic process for the preparation of olefins

(30) Priorité: 25.09.1990 FR 9011911
(43) Date de publication de la demande: 01.04.1992
(73) Titulaire: INSTITUT FRANCAIS DU PETROLE, F-92502 Rueil-Malmaison (FR)
(72) Inventeur: Burzynski, Jean-Pierre, F-69005 Lyon (FR); Hoffmann, Frédéric, F-75010 Paris (FR); Hugues, François, Charly, F-69390 Vernaison (FR); Pontier, Renaud, F-38200 Vienne (FR); Vuillemot, Daniel, F-69230 St Genis Laval (FR)

(56) Documents cités:
- EP-A- 0 340 852
- FR-A- 2 575 546
- US-A- 4 197 418
- US-A- 4 238 631
- US-A- 4 757 039
- US-A- 4 822 761

## Description

L'invention concerne un procédé de conversion d'une charge comprenant au moins un composé oxygéné et plus spécialement au moins un alcool tel que le méthanol et/ou au moins un étheroxyde tel que le diméthyléther, avec échange thermique en lit fluidisé. Plus particulièrement, le procédé peut s'appliquer à la régénération de catalyseurs particulièrement chargés en résidus hydrocarbonés et en coke après réaction avec une charge contenant au moins un composé oxygéné.

Ce procédé s'applique à la production d'hydrocarbures oléfiniques, notamment à usage pétrochimique, comprenant une majeure partie de composés ayant de 2 à 4 atomes de carbone dans leur molécule, en présence d'un catalyseur comme une zéolithe circulant dans un réacteur à lit fluidisé.

Une utilisation particulière donnée ici à titre d'exemple est la régénération de catalyseurs de conversion catalytique du méthanol en hydrocarbures riches en éthylène et/ou en propylène.

En particulier, la réaction de conversion du méthanol met en jeu deux réactions exothermiques en présence du catalyseur : la réaction de conversion de la charge proprement dite et la réaction de régénération du catalyseur sur lequel du coke et des hydrocarbures peuvent se déposer. Ces deux réactions, si elles sont mal contrôlées, peuvent provoquer un dégagement de chaleur tel qu'il peut détériorer l'appareillage et désactiver le catalyseur.

En ce qui concerne les régénérations de catalyseurs, pour pallier ces inconvénients, il a été proposé de réaliser une régénération en deux zones où la combustion fonctionne en tout ou partie à co-courant de catalyseur et de fluide oxydant de façon à minimiser le temps de séjour du catalyseur à haute température, sans toutefois dépasser 750°C, pour ne pas diminuer l'activité du catalyseur. Le recyclage d'une partie de catalyseur régénéré chaud au catalyseur usagé dans la partie inférieure du régénérateur permet d'initier la combustion des dépôts hydrocarbonés sur le catalyseur usagé et la conversion du monoxyde de carbone. La température de régénération est néanmoins limitée à 750°C en raison de la présence de vapeur d'eau, résultant de la combustion de l'hydrogène, du coke et des hydrocarbures, pouvant nuire à la stabilité du catalyseur, et en raison des limites métallurgiques propres à l'acier inox généralement utilisé.

Il est également connu d'introduire dans une zone inférieure d'un régénérateur un mélange de catalyseur régénéré chaud en provenance d'une seconde zone supérieure et de catalyseur régénéré froid pour contrôler la température de combustion du catalyseur dans le régénérateur. Pour refroidir le catalyseur régénéré, on l'introduit dans un échangeur thermique. Ce catalyseur chaud circule notamment de bas en haut sous la forme d'un lit fluidisé dense dans l'échangeur.

Comme il est nécessaire par ailleurs, pour amener ce catalyseur régénéré et refroidi dans la zone de combustion du régénérateur, de le mélanger avec du catalyseur régénéré chaud, du catalyseur usagé et de l'air de fluidisation dans une conduite de mélange en aval de l'échangeur et en amont de la zone de combustion, l'obligation de fluidisation à la sortie de l'échangeur complique énormément l'écoulement des différentes phases. Il en résulte notamment une perturbation en sortie de l'échangeur de sorte que la surface d'échange n'est pas utilisée à son maximum et le contrôle du niveau de catalyseur dans l'échangeur devient aléatoire. Par ailleurs, le risque d'érosion est augmenté. Enfin, comme l'introduction du catalyseur régénéré chaud, celle du catalyseur froid et celle du catalyseur usagé sont effectuées à la partie la plus basse du régénérateur dans une phase dense, il est nécessaire de fluidiser les grains de ces catalyseurs contenus dans le régénérateur pour procéder à la combustion en phase diluée et l'envoyer ensuite dans la seconde zone du régénérateur.

Il est envisageable de contrôler la température de régénération par dérivation d'une partie du catalyseur dans un échangeur de chaleur, placé à la base d'une zone de désengagement située à la partie supérieure du régénérateur et comportant un faisceau d'échange permettant de refroidir ladite partie de catalyseur au niveau de température requis. Dans cet échangeur, la circulation du catalyseur ne suit aucun trajet déterminé ; le flux d'échange de catalyseur entre l'échangeur et la régénération s'établit en fonction uniquement des degrés de fluidisation de ces deux récipients et des niveaux de pression dans la zone de désengagement. De ce fait, la circulation de catalyseur peut être qualifiée d'erratique en ce sens qu'elle s'établit tantôt préférentiellement du régénérateur vers l'échangeur, et tantôt préférentiellement en sens inverse.

De plus, le débit global de catalyseur s'établissant entre l'échangeur et le régénérateur n'est pas contrôlable, ce qui signifie pratiquement que ce type d'échangeur ne possède qu'un seul niveau de fonctionnement, en d'autres termes une souplesse extrêmement réduite.

Selon un procédé connu, on fait entrer dans un échangeur de chaleur dont l'entrée est à l'intérieur d'un lit dense d'un régénérateur, une partie du catalyseur usagé et on le fait sortir à un niveau plus élevé au sein du même lit dense, la remontée du catalyseur refroidi étant effectuée par la force d'ascension d'un gaz de fluidisation situé à la base de l'échangeur. Cette force d'ascension implique des vitesses de gaz très élevées (plusieurs mètres par seconde) qui perturbent la zone dense du régénérateur et rendent difficile l'entrée du catalyseur dans l'échangeur de chaleur. Il s'en suit un échange de chaleur limité.

Par ailleurs, l'art antérieur est illustré par les brevets suivants : US-A-4238 631, US-A-4197 418, US-A-4822 761, US-A-4757 039, EP-A-0340 852 et FR-A-2575 546.

Les conditions opératoires relatives aux procédés de conversion de charges oxygénées comme le méthanol sont par exemples décrites dans les brevets suivants : US-A-4689205, US-A-3969426 et US-A-4229608, mais aucun de ces brevets ne décrit le contrôle du niveau thermique de la réaction et de la régénération du catalyseur qui est un des problèmes cruciaux du procédé. L'objet de l'invention permet de remédier aux inconvénients mentionnés ci-avant.

La présente invention offre, dans l'utilisation particulière ci-dessus, un procédé simple pour contrôler la température du catalyseur dans une zone de régénération et pour maintenir le niveau thermique, par exemple dans l'unité de conversion catalytique des composés oxygénés, à un seuil acceptable.

D'une manière plus générale, l'invention permet la régulation thermique ou encore une régularisation thermique et/ou permet de régulariser ou réguler des débits de lits fluidisés.

Il s'avère possible, d'une façon particulière, d'évacuer les calories excédentaires et d'assurer la régularisation thermique des lits fluidisés conforme à l'invention dans au moins une zone de régénération. L'invention concerne donc un procédé de conversion d'une charge contenant une majeure partie d'au moins un composé oxygéné tel que le méthanol en hydrocarbures oléfiniques riches en composés ayant de 2 à 4 atomes de carbone dans leur molécule tels que l'éthylène et le propylène. Ce procédé comprend la conversion de la charge dans une zone réactionnelle de conversion dans des conditions appropriées en présence d'un catalyseur. On introduit le catalyseur en provenance de la zone réactionnelle dans au moins une zone de régénération, on effectue la régénération du catalyseur, dans des conditions appropriées, dans une zone à lit fluidisé dense, on soutire du lit fluidisé dense une partie au moins du catalyseur, de préférence par un organe de liaison, on envoie cette partie du catalyseur dans une zone de régulation ou contrôle de niveau thermique avantageusement allongée le long d'un axe de symétrie, on procède à une régulation ou contrôle de niveau thermique par échange de chaleur indirect avec un fluide et on réintroduit cette partie du catalyseur ainsi régulé thermiquement dans le lit fluidisé dense de la zone de régénération, de préférence sensiblement au même niveau, par le même organe de liaison. De manière plus précise, on fait circuler en lit fluidisé dense ladite partie du catalyseur dans la zone de régulation comportant une cloison interne de séparation qui définit deux compartiments adjacents, allongés selon leur axe de symétrie et communiquant par leur partie inférieure, le catalyseur s'écoulant de préférence de manière descendante à l'état fluidisé dans l'un des compartiments, la vitesse de fluidisation dans ce compartiment étant comprise entre 0,1 cm et 2 m/s et le catalyseur remontant à l'état fluidisé dans l'autre compartiment, la vitesse de fluidisation dans l'autre compartiment étant comprise entre 0,1 et 6 m/s et étant de préférence au moins égale à la vitesse de fluidisation dans le compartiment descendant.

La circulation organisée d'un compartiment à l'autre, avec passage dans la partie inférieure de la zone de régulation, c'est-à-dire de refroidissement, permet un contrôle du débit de catalyseur refroidi entre la zone de régénération et la zone de refroidissement (ou échangeur de chaleur) par un ajustement adéquat des vitesses de fluidisation dans chaque compartiment de la zone de refroidissement. De façon avantageuse, la vitesse de fluidisation dans le compartiment dans lequel descend le catalyseur est comprise entre 0, 1 cm/s et 1 m/s et celle dans l'autre compartiment dans lequel le catalyseur remonte refroidi pour aboutir à la base du lit fluidisé dense du régénérateur, est comprise entre 0,3 et 5 m/s. On a observé dans ces conditions une entrée facilitée du catalyseur dans l'échangeur thermique, l'absence substantielle de perturbation dans le lit dense du régénérateur et un excellent niveau d'échange thermique.

Selon une caractéristique du procédé, la zone de refroidissement comporte une cloison interne de séparation entre les deux compartiments allongés et adjacents qui communiquent par leur partie inférieure. Cette cloison peut être une surface plane qui peut s'appuyer sensiblement selon l'axe de l'échangeur sur la paroi généralement cylindrique de l'échangeur thermique, par exemple suivant une de ses génératrices, ou une enveloppe coaxiale audit axe. De manière préférée, cette cloison peut avoir une section circulaire ou rectangulaire selon un plan perpendiculaire à l'axe de l'échangeur, sensiblement coaxiale au cylindre de l'échangeur et de hauteur inférieure à celle de l'échangeur de façon à permettre la communication des compartiments concentriques ainsi définis.

Avantageusement, le compartiment où on effectue la remontée du catalyseur vers le régénérateur comporte des moyens d'échange de chaleur décrits ci-après. Dans le cas de compartiments coaxiaux, c'est de préférence le compartiment central qui contient les moyens d'échange de chaleur et par lequel on effectue la remontée du catalyseur. L'échange thermique est alors meilleur car la vitesse du catalyseur assurée par le gaz de fluidisation, habituellement de l'air, est plus importante.

Pour éviter tout problème d'érosion, la distance R selon l'axe de l'échangeur de chaleur entre la partie inférieure de la cloison et des organes d'injection d'un gaz de fluidisation dans chacun des compartiments est généralement comprise entre 0 et 0,8 m et avantageusement comprise entre 0,4 et 0,6 m. De préférence, ces organes d'injection sont disposés à l'intérieur des compartiments. La partie supérieure de la cloison ne dépasse pas, en règle générale, le niveau supérieur de l'échangeur de sorte qu'elle ne déborde sensiblement pas dans le lit dense du régénérateur. La régénération du catalyseur usagé peut être effectuée dans deux zones de régénération distinctes et séparées. Selon un premier mode de réalisation, on effectue une première régénération du catalyseur en provenance d'une zone réactionnelle dans une première zone de régénération, on refroidit le catalyseur au moins en partie régénéré selon le procédé décrit ci-avant, et on envoie le catalyseur refroidi de la première zone dans une seconde zone de régénération où l'on effectue une seconde régénération. Le catalyseur régénéré et séparé des effluents de combustion est en général recyclé de la seconde zone de régénération vers la zone reactionnelle.

Selon un second mode de réalisation, on effectue une première régénération du catalyseur en provenance de la zone réactionnelle dans une première zone de régénération, on envoie le catalyseur au moins en partie régénéré vers une seconde zone de régénération, on effectue une seconde régénération, on refroidit le catalyseur selon le procédé ci-avant et on recycle le catalyseur régénéré et séparé des effluents de combustion de la seconde zone de régénération vers la zone réactionnelle.

Les effluents de première régénération peuvent soit être séparés dans la première zone de régénération, soit envoyés avec le catalyseur vers la seconde zone de régénération où il sont ensuite séparés.

Tous ces moyens combinés entre eux coopèrent à l'obtention d'un meilleur échange thermique par rapport à l'art antérieur dans un dispositif à coût réduit et à utilisation simplifiée.

L'échangeur de chaleur peut être d'un type connu en soi. Il peut comprendre une calandre d'orientation verticale dans laquelle le catalyseur peut par exemple circuler à travers l'extérieur des tubes de refroidissement, le fluide de refroidissement, en général de l'eau, circulant à l'intérieur des tubes.

Selon un autre mode de réalisation, l'échangeur de chaleur peut comprendre une pluralité de tubes de forme sinueuse disposés selon son axe de symétrie. Par forme sinueuse, on entend une forme sinusoïdale, crénelée, en zigzag, en accordéon.

Selon un autre mode de réalisation, l'échangeur peut être d'un autre type : la cloison délimitant les compartiments de l'échangeur peut représenter une partie de la surface d'échange. Cette surface est réalisée sous forme de tubes-membranes : une pluralité de tubes où circule le fluide de refroidissement, et s'étendant selon l'axe longitudinal de l'échangeur, est reliée par des ailettes soudées longitudinalement de manière à former une surface continue étanche à la pression. La surface d'échange n'étant en général pas suffisante pour assurer l'intégralité de l'échange de chaleur requis, l'intérieur du compartiment où s'effectue le refroidissement du catalyseur peut être rempli d'une pluralité de tubes d'échange disposés comme indiqué ci-avant. Cette solution présente l'avantage d'augmenter la surface d'échange pour un volume de l'échangeur thermique donné, notamment lorsque la cloison entoure les moyens d'échange de chaleur.

Un dispositif pour la régénération en lit fluidisé et en continu d'un catalyseur usagé peut comprendre en combinaison :
a) une unité de régénération (I) sensiblement verticale dont la partie inférieure comporte un lit (3) fluidisé dense du catalyseur,
b) un échangeur de chaleur (6) de forme allongée, de préférence cylindrique et disposé avantageusement de manière sensiblement verticale, ayant un axe de symétrie et pouvant avoir, éventuellement, à sa partie supérieure une entrée et une sortie de catalyseur qui communiquent avec ledit lit dense de préférence sensiblement au même niveau,
c) des moyens d'échange de chaleur (25) contenus dans ledit échangeur de chaleur, reliés à des conduites d'entrée (26) et de sortie (27) d'un fluide de régulation thermique.

Cet échangeur comprend par ailleurs en combinaison :
- une cloison interne de séparation (22) définissant deux compartiments adjacents allongés (23, 24) suivant ledit axe de symétrie, un premier compartiment ayant une extrémité supérieure reliée à ladite entrée de catalyseur dans le compartiment et éventuellement une extrémité inférieure par laquelle sort le catalyseur, un second compartiment ayant une extrémité inférieure par laquelle éventuellement entre le catalyseur en provenance du premier compartiment, et une extrémité supérieure reliée à ladite sortie éventuelle de catalyseur,
- un espace de passage (30) adapté au passage du catalyseur du premier compartiment au second compartiment,
- des premiers moyens d'injection (28) d'un gaz de fluidisation dans le premier compartiment, disposés au voisinage de l'extrémité inférieure du premier compartiment et adaptés à faire éventuellement s'écouler le catalyseur de manière descendante à l'état fluidisé et,
- des seconds moyens d'injection (31) d'un gaz de fluidisation dans le second compartiment, disposés au voisinage de l'extrémité inférieure du second compartiment et adaptés à faire s'écouler le catalyseur de manière ascendante à l'état fluidisé ; cette injection peut être purement verticale ou provoquer un courant ascendant hélicoïdal selon un pas et une vitesse désirés, et éventuellement,
- un diffuseur généralement dans la partie inférieure de l'échangeur d'un fluide généralement gazeux pour assurer la fluidisation du catalyseur.

Selon une autre caractéristique du dispositif, ce dernier peut comporter des moyens de contrôle de débit du gaz de fluidisation reliés auxdits moyens d'injection de ce gaz et asservis par des moyens d'asservissement à un moyen de mesure de la température dans l'unité de régénération. Lorsque le dispositif comporte deux unités de régénération, le moyen de mesure de la température peut se trouver soit dans la première unité, soit dans la seconde.

Selon une autre caractéristique du dispositif selon l'invention, le dispositif peut comprendre un organe de liaison entre l'unité de régénération et l'échangeur de chaleur, avec un axe de symétrie P orienté selon un angle A par rapport à l'axe de symétrie de l'échangeur compris en général entre 0 et 80°, avantageusement entre 40 et 50° et de diamètre Q compris entre 0,8 et 1,5 fois, de préférence entre 0,9 et 1,2 fois le diamètre D de l'échangeur (enveloppe externe). Il peut comprendre au moins un organe d'aération par un gaz tel que de l'air disposé sensiblement au voisinage de l'axe P de l'organe de liaison et adapté à réaliser une vitesse de jet rapportée à la section de l'organe d'aération comprise entre 50 et 150 m/s et de préférence entre 80 et 120 m/s.

Selon un autre mode de mise en oeuvre du procédé, l'organe de liaison peut comporter une cloison interne qui définit deux chambres dont l'une communique avec le premier compartiment de l'échangeur et dont l'autre communique avec le second compartiment. Il comporte en outre au moins un organe d'aération disposé sensiblement selon l'axe P de l'organe de liaison et de préférence au voisinage de l'axe P, dans la chambre communiquant avec le compartiment ou s'effectue la remontée du catalyseur.

Dans le cadre de la présente invention, il n'est pas mentionné le type de catalyseurs employé, ni les divers fluides de fluidisation qui sont des données classiques bien connues de l'homme du métier.

On utilise de préférence des catalyseurs zéolithiques et de la vapeur d'eau, au moins un gaz inerte ou des hydrocarbures gazeux comme fluide de fluidisation.

Les conditions opératoires de la conversion de la charge oxygénée sont en général les suivantes :
- la charge, qui le plus souvent contient un alcool et habituellement le méthanol, renferme généralement de 50 à 100 %, de préférence de 70 à 90 % en poids de composés oxygénés et de 0 à 50 %, de préférence de 10 à 30 % en poids d'eau. Dans le cas le plus fréquent où la charge contient du méthanol, elle peut être constituée par du méthanol brut tel que par exemple du méthanol provenant d'une unité de synthèse de ce composé à partir d'un gaz contenant du monoxyde de carbone,
- la température d'injection est habituellement égale à la température de rosée de la charge,
- la pression absolue régnant dans l'unité de conversion est habituellement de 0,1 à 0,5 mégapascal (MPa) et le plus souvent d'environ 0,2 MPa,
- la température de la zone réactionnelle est habituellement de 500 à 620 °C et le plus souvent de 550 à 590 °C,
- le temps de séjour dans la zone réactionnelle est habituellement de 0,05 à 10 secondes et le plus souvent de 0,5 à 3 secondes,
- la vitesse des gaz dans la zone réactionnelle est habituellement de 5 à 30 m/s et le plus souvent de 10 à 20 m/s, et
- la température de régénération est habituellement de 500 à 750 °C et le plus souvent de 600 à 650 °C.

Les conditions opératoires sont choisies pour que la composition des produits obtenus, exprimée par le rendement en carbone en composés éthyléniques ayant de 2 à 4 atomes de carbone dans leur molécule, soit telle que le rendement soit supérieur à 70 % et de préférence supérieur à 80 %. Les produits obtenus peuvent être particulièrement riches en éthylène (rendement en carbone pour l'éthylène supérieur à 40 %) ou particulièrement riches en propylène (rendement en carbone pour le propylène supérieur à 60 %).

Les figures ci-dessous illustrent de façon schématique la mise en oeuvre d'un procédé de régénération utilisé dans le cadre d'une unité de conversion catalytique en lit fluidisé, d'une charge contenant au moins un alcool, avec double régénération du catalyseur, la deuxième unité de régénération se trouvant au-dessus de la première selon un même axe vertical. Parmi celles-ci,
- la figure 1 montre un dispositif où le catalyseur régénéré dans un premier régénérateur circule vers un échangeur thermique avant d'être régénéré dans un second régénérateur,
- la figure 2 schématise l'échangeur thermique en coupe longitudinale avec un organe de liaison communiquant avec le premier régénérateur et un faisceau d'échange préféré,
- la figure 3 montre l'échangeur thermique en coupe longitudinale comprenant l'organe de liaison qui comporte une cloison définissant deux chambres coaxiales, et
- la figure 4 représente une coupe longitudinale de l'échangeur thermique montrant un faisceau de tubes.

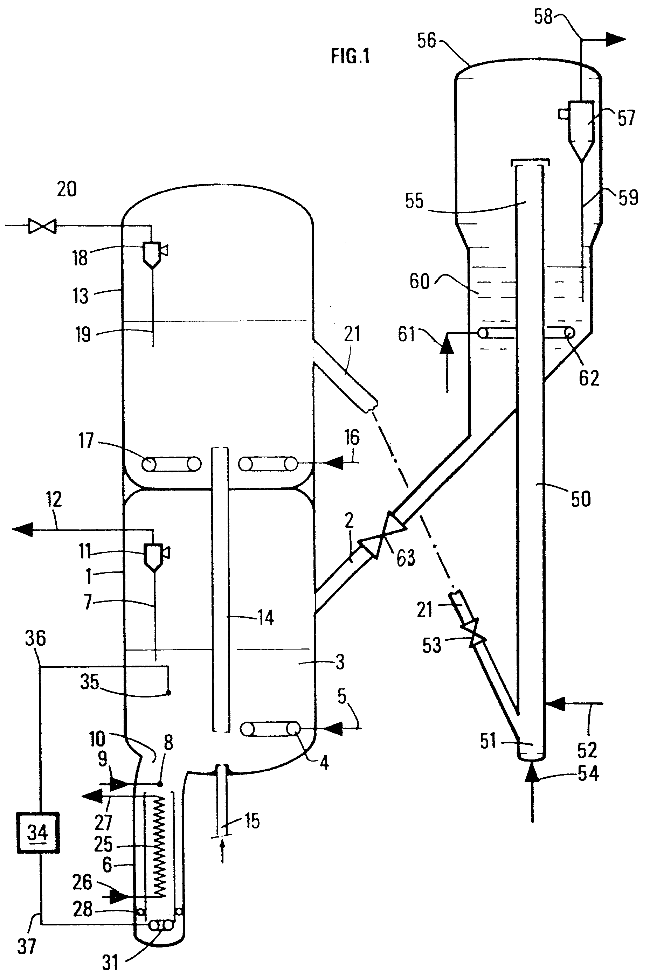

Le dispositif de la figure 1 comprend un élévateur (ou riser) (50) allongé, sensiblement vertical, à l'extrémité inférieure (51) duquel est injectée la charge comprenant du méthanol, de préférence gazeux, par des injecteurs appropriés (52). Du catalyseur, par exemple une zéolithe, au moins en partie régénéré dans un régénérateur (13) arrive par une ligne (21), à l'état fluidisé, à l'extrémité inférieure de l'élévateur (50) où il est mis en contact avec la charge. Cette ligne (21) est contrôlée par une vanne (53).

Des moyens appropriés de fluidisation (54), par un gaz tel que de la vapeur d'eau, permettent de maintenir la suspension catalyseur-charge à l'état fluidisé. La mise en contact dans l'élévateur du catalyseur et de la charge s'effectue à une température voisine de 550 °C pendant un temps de séjour dans le lit fluidisé d'environ 2 à 5 secondes, par exemple, et on obtient un mélange d'hydrocarbures riche en composés éthyléniques.

Le gaz de fluidisation est animé d'une vitesse telle que la masse volumique de la suspension dans la zone réactionnelle est d'environ 100 kg/m³ et sa vitesse d'environ 15 m/s. Le catalyseur et l'effluent de la réaction de conversion sont séparés à l'extrémité supérieure (55) de l'élévateur dans une zone de séparation (56) et au moins un cyclone (57) interne ou externe évacue l'effluent produit par une ligne (58), tandis que les particules catalytiques entraînées sont réintroduites par une jambe (59) du cyclone dans le lit fluide dense (60) du séparateur. Une séparation secondaire par strippage (62) à la vapeur d'eau amenée par une ligne (61) permet d'améliorer la séparation des particules et de l'effluent dans la partie inférieure du séparateur. Celle-ci est reliée au régénérateur (1) d'une unité de régénération qui en comporte deux (1, 13) par une ligne (2). Celle-ci, contrôlée par une vanne (63), conduit le catalyseur usagé chargé de coke dans le lit fluidisé dense (3) à la partie inférieure du premier régénérateur (1). Ce lit fluidisé est réalisé par un anneau ou courone de fluidisation (4) à l'aide d'un gaz, de l'air par exemple, amené par une ligne (5).

Selon la figure 1, le catalyseur au moins en partie régénéré de préférence à contre-courant par l'air ci-dessus tombe par gravité vers un échangeur thermique (6), avantageusement vertical, dont l'entrée et la sortie sont connectées à la base du premier régénérateur (1) par l'intermédiaire d'un organe de liaison (10) assurant le passage du catalyseur avant et après refroidissement. Des moyens d'aération (8) par de l'air amené par une ligne (9) aérent le catalyseur dans l'organe de liaison (10).

Une fois refroidi selon le procédé de l'invention décrit ci-avant, il remonte dans le lit fluidisé dense du premier régénérateur par le même organe de liaison (10) et sensiblement au même niveau. Les particules du catalyseur sont entraînées par le gaz de combustion et séparées par des cyclones (11) internes, disposés avantageusement dans la partie supérieure de la première unité de régénération. Le gaz de combustion riche en oxyde de carbone et en eau est évacué par une ligne (12) sous pression pour une régénération ultérieure, tandis que les particules de catalyseur retombent par la jambe (7) vers la base du premier régénérateur (1). Elles sont ensuite transférées à la seconde unité de régénération par le conduit (14) alimenté en air par la ligne (15).

La base de la seconde unité de régénération est également alimentée en air par la ligne (16) et les injecteurs (17). La combustion du coke restant s'effectue à contre-courant de l'air injecté.

Les gaz de combustion évacués à la partie supérieure du second régénérateur (13) sont traités dans un cyclone intérieur ou extérieur (18), à la base duquel les particules du catalyseur sont retournées par le conduit (19) au second régénérateur (13), tandis que les gaz de combustion sont évacués par la ligne (20) où une vanne de sécurité est prévue.

Les particules catalytiques régénérées et à la température optimale souhaitée (600 °C environ) sont recyclées à débit contrôlé par un conduit de recyclage (21) à l'alimentation de l'élévateur (50) de l'unité de conversion de la charge.

Bien qu'on puisse entraîner de la chaleur à n'importe quel point de l'unité de conversion, il est avantageux de l'entraîner au niveau de l'un des régénérateurs et il est préférable, notamment pour des raisons pratiques, de disposer l'échangeur thermique (6) au niveau du premier régénérateur (1).

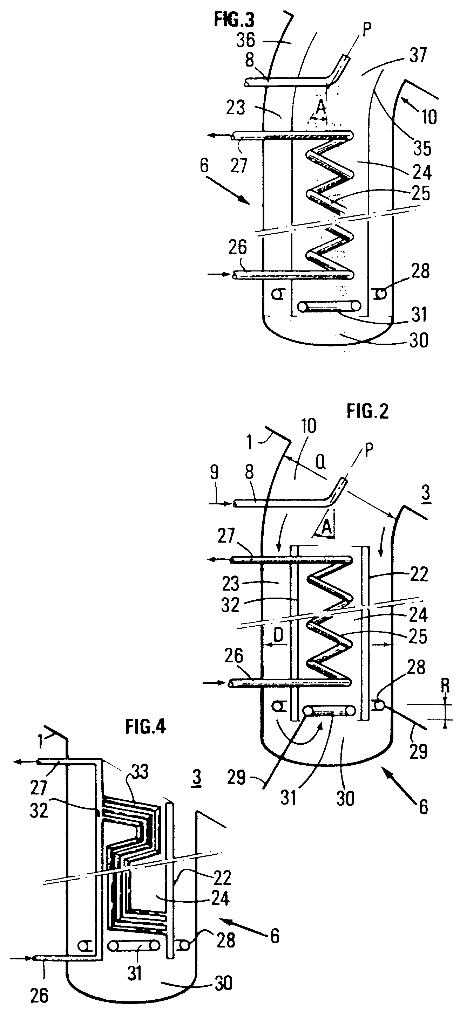

Une partie au moins du catalyseur, au moins en partie régénéré, est soutirée du lit dense (3) et descend selon la figure 2 dans l'échangeur thermique. Celui-ci, de forme allongée, par exemple cylindrique, comprend une cloison interne de séparation (22) formant un cylindre sensiblement co-axial à l'échangeur qui le contient. Cette cloison délimite deux compartiments allongés, adjacents (23) et (24), dont l'un d'eux est de forme annulaire et l'autre, central, de forme cylindrique ; ces deux compartiments ont donc une paroi commune, la cloison ci-dessus.

La surface du compartiment central définie par un plan sensiblement perpendiculaire à l'axe de l'échangeur et de forme avantageusement circulaire est en général comprise entre 0,2 et 0,7 fois la surface circulaire de l'échangeur correspondant à son enveloppe externe et définie par le même plan.

Ce compartiment central contient des moyens d'échange de chaleur (25), de forme appropriée entourés par la cloison (22) ; leur partie inférieure est alimentée par exemple par de l'eau de refroidissement ou tout autre fluide, huile etc. amené par une ligne (26) et la partie supérieure ou sortie de ces moyens (25) évacue un mélange biphasique d'eau et de vapeur correspondant à l'échange par une ligne (27).

Les deux compartiments communiquent entre eux par leur partie inférieure : la cloison de séparation détermine en effet un espace de passage (30) pour le catalyseur puisque cette cloison n'atteint pas l'extrémité basse de l'échangeur. La position de la cloison est détérminée par la distance R de son extrémité inférieure à des moyens de fluidisation ou d'injection (28) et (31) d'un gaz tel que de l'air, amené par une ligne (29), et disposés au voisinage de la partie inférieure des compartiments et de préférence à l'intérieur de chacun des compartiments. Cette distance est avantageusement comprise entre 0,4 et 0,6 mètre.

Le catalyseur circule du haut vers le bas dans le compartiment périphérique, en lit fluidisé. Il est animé par des premiers moyens de fluidisation (28) (un anneau ou une grille) qui sont adaptés à délivrer dans ce compartiment périphérique une vitesse de fluidisation comprise par exemple entre 1 cm/s et 10 cm/s. Il traverse ensuite l'espace de passage (30) à la base de l'échangeur et remonte dans le compartiment central en lit fluidisé. Des seconds moyens de fluidisation (31) (anneau ou grille) sont en effet adaptés à délivrer, dans le compartiment central, de volume généralement plus important, une vitesse de fluidisation par exemple comprise entre 0,1 m/s et 1 m/s.

La cloison interne de séparation entourant les moyens d'échange peut être constituée par une pluralité de tubes membranes (32) formant l'enveloppe du compartiment central et à travers lesquels circule le fluide de refroidissement. Ces tubes s'étendent de manière sensiblement parallèle à l'axe longitudinal de l'échangeur et sont reliés par des ailettes soudées longitudinalement de manière à constituer ladite enveloppe.

Les moyens d'échange de chaleur (25) à l'intérieur du compartiment central peuvent être un faisceau de tubes (33) répartis régulièrement autour de l'axe de l'échangeur. De manière préférée, comme le montre la figure 4, le faisceau est constitué d'une pluralité de tubes (33) de forme sinueuse, crénelée, à angles non droits définissant des nappes s'imbriquant les unes dans les autres et disposés sensiblement selon l'axe de l'échangeur. La distance entre nappes est généralement comprise entre 4 et 7 fois le diamètre du tube.

De manière générale, la partie supérieure de la cloison de séparation ne dépasse sensiblement pas le prolongement de la paroi inférieure du régénérateur sur laquelle est inséré l'échangeur (fig. 4).

Dans le cas où l'échangeur possède avantageusement un organe de liaison (10) (fig. 2) assurant la communication du catalyseur entre l'échangeur proprement dit et le régénérateur, la cloison atteint généralement le niveau supérieur de la partie cylindrique (fig. 2) de l'échangeur.

L'organe de liaison (10) d'axe de symétrie P est généralement orienté selon un angle A par rapport à l'axe de symétrie de l'échangeur compris entre 0 et 80°, de préférence entre 40 et 50° et son diamètre extérieur Q est habituellement compris entre 0,8 et 1,5 fois le diamètre extérieur D de l'échangeur et de préférence entre 0,9 et 1,2 fois. Le cas où l'angle A est égal à zéro est illustré par la figure 4. A l'intérieur de l'organe de liaison (10), des moyens d'aération (9) avantageusement disposés au voisinage de l'axe de symétrie P, dirigent de l'air d'aération en direction du régénérateur à une vitesse de jet rapportée à la section des moyens d'aération comprise entre 50 et 150 m/s et avantageusement comprise entre 80 et 120 m/s.

Selon la figure 3 représentant un autre mode de réalisation du dispositif, celui-ci comprend un organe de liaison (10) entre l'unité de régénération (1) et l'échangeur de chaleur (6), ayant un axe de symétrie P orienté selon un angle A par rapport à l'axe de symétrie de l'échangeur compris entre 0 et 80°. Cet organe (10) comporte une cloison (35) de section circulaire, disposée sensiblement selon l'axe P de l'organe de liaison, qui définit deux chambres (36) et (37) sensiblement coaxiales dont l'une (36), annulaire, communique avec le compartiment (23) où descend le catalyseur et dont l'autre (37) communique avec le compartiment (24) central où remonte le catalyseur. En outre, la chambre (37) où sont répartis les tubes d'échange (33) comprend au moins un organe d'aération (8) du catalyseur disposé sensiblement au voisinage de l'axe P de l'organe de liaison.

Le dispositif schématisé sur les figures comporte un moyen de contrôle (34) de débit du gaz de fluidisation relié au moyens d'injection (28, 31) de ce gaz et avantageusement au moyen d'injection (31) dans le compartiment central où s'effectue la remontée du catalyseur. Ce moyen de contrôle (34) est asservi à un moyen de mesure (35) de la température du catalyseur dans le premier régénérateur (1) ou éventuellement dans le second régénérateur (13) grâce à des lignes de liaison (36) et (37).

On ajuste le débit de catalyseur circulant dans l'échangeur thermique en agissant sur la vitesse de fluidisation dans le compartiment où s'effectue une circulation ascendante pour maintenir la température du premier ou du second régénérateur à un niveau satisfaisant et donc la température du catalyseur régénéré devant être recyclé à l'entrée de l'élévateur de la zone réactionnelle, à une température de consigne qui dépend de la charge à convertir.

Lorsque la température du régénérateur est supérieure à la température de consigne, le moyen de contrôle envoie un signal au moyen d'injection (31) d'air de fluidisation de façon à augmenter la vitesse de circulation dans le compartiment central de refroidissement. Au contraire, lorsque la température de régénération est inférieure à la température de consigne, le moyen de contrôle (34) émet un signal qui agit sur le moyen d'injection (31) d'air et qui permet de diminuer la vitesse de fluidisation dans le compartiment central, voire de stopper l'échange. Le tableau 1 ci-après illustre le niveau d'échange thermique en fonction de la vitesse de fluidisation dans le compartiment central de remontée du catalyseur.

**Tableau I**

| Vitesse de fluidisation (m/s) | % de l'échange maximal |
|---|---|
| 1,1 | 100 |
| 0,7 | 100 |
| 0,4 | 50 |
| 0,1 | 20 |
| 0,0 | 0 |

Des essais ont montré qu'en opérant selon le procédé de l'invention dans les conditions suivantes, en présence d'un catalyseur zéolithique :
- vitesse de fluidisation dans la zone de régénération : 0,6 m/s
- vitesse de fluidisation dans la partie descendante de l'échangeur : 0,15 m/s
- vitesse de fluidisation dans la partie ascendante de l'échangeur : 1,1 m/s
on obtenait un gain de 35% sur la valeur maximale de l'échange.

On a illustré et décrit le cas où le compartiment central était cylindrique. L'invention resterait la même si le compartiment central avait une section rectangulaire. L'invention resterait également la même avec une cloison plane définissant deux compartiments allongés adjacents.

## Revendications

1. Procédé de conversion d'une charge contenant une majeure partie d'au moins un composé oxygéné en hydrocarbures oléfiniques riches en composés ayant de 2 à 4 atomes de carbone dans leur molécule comprenant la conversion en lit fluide de ladite charge dans une zone réactionnelle de conversion dans des conditions appropriées en présence d'un catalyseur, le procédé comprenant au moins une zone de régénération dans laquelle on introduit ledit catalyseur en provenance de ladite zone réactionnelle, on effectue une régénération du catalyseur dans une zone à lit fluidisé dense en présence d'un gaz contenant de l'oxygène dans des conditions de régénération appropriées, on soutire du lit fluidisé dense une partie au moins du catalyseur, on envoie cette partie du catalyseur dans une zone de régulation ou contrôle de niveau thermique avantageusement allongée le long d'un axe de symétrie, on procède à une régulation ou contrôle de niveau thermique par échange de chaleur indirect avec un fluide, on réintroduit cette partie du catalyseur ainsi régulé thermiquement dans ledit lit fluidisé dense de la zone de régénération, de préférence sensiblement au même niveau,
le procédé étant caractérisé en ce qu'on fait circuler en lit fluidisé dense ladite partie du catalyseur dans la zone de régulation (6) comportant une cloison interne de séparation (22) qui définit deux compartiments adjacents, allongés selon ledit axe de symétrie et communiquant par leur partie inférieure (30), le catalyseur s'écoulant de manière descendante à l'état fluidisé dans l'un des compartiments, la vitesse de fluidisation dans ce compartiment étant comprise entre 0,1 cm/s et 2 m/s et le catalyseur remontant à l'état fluidisé dans l'autre compartiment, la vitesse de fluidisation dans l'autre compartiment étant comprise entre 0,1 et 6 m/s et étant de préférence au moins égale à la vitesse de fluidisation dans le compartiment descendant.

2. Procédé selon la revendication 1 dans lequel la vitesse de fluidisation dans le compartiment où descend le catalyseur est comprise entre 0,1 cm/s et 1 m/s et la vitesse de fluidisation dans le compartiment où remonte le catalyseur est comprise entre 0,3 et 5 m/s.

3. Procédé selon la revendication 1 ou 2 dans lequel ladite cloison de séparation définit deux compartiments coaxiaux.

4. Procédé selon l'une des revendications 1 à 3 comprenant deux zones de régénération dudit catalyseur usagé, caractérisé en ce qu'on effectue une première régénération du catalyseur dans une première zone de régénération et en ce que, soit on refroidit ledit catalyseur selon l'une des revendications 1 à 2 et on envoie le catalyseur refroidi au moins en partie régénéré de la première zone de régénération vers une seconde zone de régénération où on effectue une seconde régénération, soit on envoie le catalyseur au moins en partie régénéré dans une seconde zone de régénération, on effectue une seconde régénération et on refroidit ledit catalyseur selon l'une des revendications 1 à 2.

5. Procédé selon l'une des revendications 1 à 4 dans lequel la charge est introduite à l'état gazeux.

6. Procédé selon l'une des revendications 1 à 5 dans lequel on soutire du lit fluidisé dense ladite partie de catalyseur par un organe de liaison et on réintroduit le catalyseur une fois refroidi dans le lit fluidisé dense de la zone de régénération par le même organe de liaison.

7. Procédé selon la revendication 6 dans lequel on aère l'organe de liaison par un organe d'aération dans des conditions telles que la vitesse de jet est de 50 à 150 m/s.

8. Procédé selon l'une des revendications 1 à 7 dans lequel on contrôle et on régule dans des conditions appropriées le débit de fluidisation par des moyens adéquats que l'on asservit à la mesure de la température de la zone de régénération.

9. Procédé selon l'une des revendications 1 à 8 dans lequel la charge comprend au moins un alcool, de préférence le méthanol.

10. Procédé selon l'une des revendications 1 à 9 dans lequel la charge contient de l'eau.

## Claims

1. A process for the conversion of a charge containing a major part of at least one oxygenated compound into olefinic hydrocarbons which are rich on compounds having from 2 to 4 carbon atoms in their molecule comprising conversion of said charge in a fluidised bed in a conversion reaction zone under suitable conditions in the presence of a catalyst, the process comprising at least one regeneration zone into which said catalyst coming from said reaction zone is introduced, regeneration of the catalyst is effected In a zone with a dense fluidised bed in the presence of a gas containing oxygen under suitable regeneration conditions, a part at least of the catalyst is withdrawn from the dense fluidised bed, said part of the catalyst is passed into a thermal level control or regulation zone which is advantageously elongate along an axis of symmetry, thermal level control or regulation is effected by indirect heat exchange with a fluid, and said part of the catalyst which is thus thermally regulated is re-introduced into said dense fluidised bed of the regeneration zone, preferably substantially at the sane level,
the process being characterised in that said part of the catalyst is circulated in a dense fluidised bed in the regulation zone (6) comprising an internal separation partition (22) which defines two adjacent compartments which are elongate along said axis of symmetry and communicate by way of their lower part (30), the catalyst flowing in a descending flow in the fluidised state in one of the compartments, the speed of fluidisation in said comportment being between 0.1 cm/s and 2 m/s and the catalyst rising in the fluidised state in the other comportment, the speed of fluidisation in the other comportment being between 0.1 and 6 m/s and preferably being at least equal to the speed of fluidisation in the descending compartment.

2. A process according to claim 1 wherein the speed of fluidisation in the comportment in which the catalyst descends is between 0.1 cm/s and 1 m/s and the speed of fluidisation in the comportment in which the catalyst rises is between 0.3 and 5 m/s.

3. A process according to claim 1 or claim 2 wherein said separation partition defines two coaxial compartments.

4. A process according to one of claims 1 to 3 comprising two zones for regeneration of said used catalyst, characterised by effecting a first step for regeneration of the catalyst in a first regeneration zone and that either said catalyst is cooled in accordance with one of claims 1 and 2 and the cooled at least partly regenerated catalyst is passed from the first regeneration zone to a second regeneration zone in which a second regeneration step is effected, or the catalyst which is at least in part regenerated is passed into a second regeneration zone, a second regeneration step is effected and said catalyst is cooled in accordance with one of claims 1 and 2.

5. A process according to one of claims 1 to 4 wherein the charge is introduced in the gaseous state.

6. A process according to one of claims 1 to 5 wherein said part of the catalyst is withdrawn fro the dense fluidised bed by a connecting member and the catalyst once cooled is re-introduced into the dense fluidised bed of the regeneration zone by the same connecting member.

7. A process according to claim 6 wherein the connecting member is aerated by an aeration member under conditions such that the jet speed is from 50 to 150 m/s.

8. A process according to one of claims 1 to 7 wherein the fluidisation rate is controlled and regulated under suitable conditions by appropriate means which are controlled in dependence on measurement of the temperature of the regeneration zone.

9. A process according to one of claims 1 to 8 wherein the charge comprises at least one alcohol, preferably methanol.

10. A process according to one of claims 1 to 9 wherein the charge contains water.

## Patentansprüche

1. Verfahren zur Umwandlung einer einen größeren Teil wenigstens einer sauerstoffhaltigen Verbindung enthaltenden Charge in olefinische Kohlenwasserstoffe, die reich an Verbindungen mit 2 bis 4 Kohlenstoffatomen in ihrem Molekül sind, die Umwandlung im Fluidbett dieser Charge in einer Reaktionsumwandlungszone unter geeigneten Bedingungen in Anwesenheit eines Katalysators umfassend, wobei das Verfahren wenigstens eine Regenerationszone umfaßt, in die man diesen aus dieser Reaktionszone stammenden Katalysator einführt, man eine Regeneration des Katalysators in einer Zone mit dichtem fluidisierten Bett in Anwensenheit eines Gases durchführt, das Sauerstoff unter geeigneten Regenerationsbedingungen enthält, man aus dem fluidisierten dichten Bett wenigstens einen Teil des Katalysators abzieht, man diesen Teil des Katalysators in eine Einstell- oder Regelzone für das thermische Niveau gibt, wobei diese Zone vorzugsweise länglich längs einer Symmetrieachse ist, man dann zu einer Einstellung oder Regelung des thermischen Niveaus durch indirekten Wärmeaustausch mit einem Fluid schreitet, man diesen Teil des so thermisch gesteuerten Katalysators in dieses fluidisierte dichte Bett der Regenerationszone, bevorzugt im wesentlichen auf dem gleichen Niveau, wieder einführt, **dadurch gekennzeichnet,** daß man im dichten fluidisierten Bett diesen Teil des Katalysators in der Steuerzone (6) zirkulieren läßt, die eine innere Trennwand (22) umfaßt, die zwei benachbarte Kammern, die längs der Symmetrieachse länglich sind, definiert und die über ihren unteren Teil (30) in Verbindung stehen, wobei der Katalysator in absteigender Weise im fluidisierten Zustand in einer der Kammern strömt, wobei die Fluidisierungsgeschwindigkeit in dieser Kammer zwischen 0,1 cm/s und 2 m/s beträgt und der Katalysator im fluidisierten Zustand in der anderen Kammer hochsteigt, wobei die Fluidisierungsgeschwindigkeit in der anderen Kammer zwischen 0,1 und 6 m/s beträgt und bevorzugt wenigstens gleich der Fluidisierungsgeschwindigkeit in der absteigenden Kammer ist.

2. Verfahren nach Anspruch 1, bei dem die Fluidisierungsgeschwindigkeit in der Kammer, wo der Katalysator nach unten geht, zwischen 0,1 cm/s und 1 m/s beträgt und die Fluidisierungsgeschwindigkeit in der Kammer, wo der Katalysator nach oben geht, zwischen 0,3 und 5 m/s ist.

3. Verfahren nach Anspruch 1 oder 2, bei dem diese Trennwand zwei koaxiale Kammern definiert.

4. Verfahren nach einem der Ansprüche 1 bis 3, zwei Regenerationszonen für diesen verbrauchten Katalysator umfassend, **dadurch gekennzeichnet,** daß man eine erste Regeneration des Katalysators in einer ersten Regenerationszone durchführt und daß man entweder diesen Katalysator nach einem der Ansprüche 1 und 2 kühlt und den gekühlten wenigstens zum Teil regenerierten Katalysator aus der ersten Regenerationszone gegen eine zweite Regenerationszone schickt, wo man eine zweite Regeneration vornimmt, oder den wenigstens zum Teil regenerierten Katalysator in eine zweite Regenerationszone gibt, eine zweite Regeneration durchführt und diesen Katalysator nach einem der Ansprüche 1 bis 2 kühlt.

5. Verfahren nach einem der Ansprüche 1 bis 4, bei dem die Charge im gasförmigen Zustand eingeführt wird.

6. Verfahren nach einem der Ansprüche 1 bis 5, bei dem man aus dem fluidisierten dichten Bett diesen Teil des Katalysators über ein Verbindungsorgan absaugt und den einmal gekühlten Katalysator in das dichte fluidisierte Bett der Regenerationszone durch das gleiche Verbindungsorgan wieder einführt.

7. Verfahren nach Anspruch 6, bei dem man das Verbindungsorgan durch ein Belüftungsorgan unter Bedingungen derart belüftet, daß die Strahlgeschwindigkeit bei 50 bis 150 m/s liegt.

8. Verfahren nach einem der Ansprüche 1 bis 7, bei dem man unter geeigneten Bedingungen den Fluidisierungsdurchsatz durch adäquate Mittel regelt und einstellt, die man bemessen auf die Temperatur der Regenerationszone zuordnet oder steuert.

9. Verfahren nach einem der Ansprüche 1 bis 8, bei dem die Charge wenigstens einen Alkohol, bevorzugt Methanol, umfaßt.

10. Verfahren nach einem der Ansprüche 1 bis 9, bei dem die Charge Wasser enthält.
